(19) Europäisches Patentamt
European Patent Office
Office européen des brevets

(11) EP 1 144 413 B1

(12) **FASCICULE DE BREVET EUROPEEN**

(45) Date de publication et mention de la délivrance du brevet:
**17.03.2004 Bulletin 2004/12**

(21) Numéro de dépôt: **99973293.6**

(22) Date de dépôt: **01.12.1999**

(51) Int Cl.[7]: **C07D 487/08**, A61K 31/44, A61K 31/501 // (C07D487/08, 209:00), C07D209:00

(86) Numéro de dépôt international:
**PCT/FR1999/002974**

(87) Numéro de publication internationale:
**WO 2000/034284 (15.06.2000 Gazette 2000/24)**

(54) **DERIVES DE 2,5-DIAZABICYCLO 2.2.1]HEPTANE, LEUR PREPARATION ET LEUR APPLICATION EN THERAPEUTIQUE**

2,5-DIAZABICYCLO(2.2.1)HEPTANDERIVATE, DEREN HERSTELLUNG UND DEREN VERWENDUNG IN HEILKUNDE

2,5-DIAZABICYCLO 2.2.1]HEPTANE DERIVATIVES, THEIR PREPARATION AND THERAPEUTIC USES

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **04.12.1998 FR 9815325**

(43) Date de publication de la demande:
**17.10.2001 Bulletin 2001/42**

(73) Titulaire: **SANOFI-SYNTHELABO**
**75013 Paris (FR)**

(72) Inventeurs:
- **LOCHEAD, Alistair**
  **F-94220 Charenton (FR)**
- **JEGHAM, Samir**
  **F-34980 Montferrier sur Lez (FR)**
- **NEDELEC, Alain**
  **F-92700 Colombes (FR)**
- **JEUNESSE, Jean**
  **F-94500 Champigny sur Marne (FR)**
- **GALLI, Frédéric**
  **F-92420 Vaucresson (FR)**
- **EVEN, Luc**
  **F-75015 Paris (FR)**

(74) Mandataire: **Ludwig, Jacques et al**
**Sanofi-Synthélabo**
**Service Brevets**
**174, avenue de France**
**75013 Paris (FR)**

(56) Documents cités:
**EP-A- 0 400 661** **US-A- 5 478 939**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 144 413 B1

Printed by Jouve, 75001 PARIS (FR)

## Description

Les composés de la présente invention répondent à la formule générale (I)

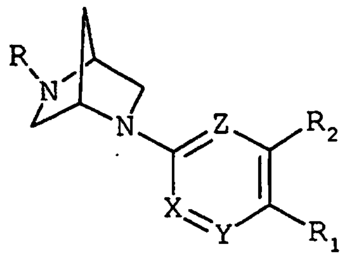

(I)

dans laquelle
l'un des symboles X, Y et Z représente un atome d'azote, un autre représente un groupe de formule C-$R_3$ et le troisième représente un atome d'azote ou un groupe de formule C-$R_4$,

$R_3$ et $R_4$ représentent chacun, indépendamment l'un de l'autre, un atome d'hydrogène ou d'halogène ou un groupe trifluorométhyle, cyano, hydroxy, ($C_1$-$C_6$)alkyle ou ($C_1$-$C_6$) alcoxy,
$R_1$ et $R_2$ représentent chacun, indépendamment l'un de l'autre, un atome d'hydrogène ou d'halogène ou un groupe trifluorométhyle, cyano, hydroxy, ($C_1$-$C_6$)alkyle, ($C_1$-$C_6$)-alcoxy, ou phényle éventuellement substitué par un ou deux atomes d'halogènes, par un ou deux groupes trifluorométhyle, par un groupe cyano, par un groupe nitro, par un groupe hydroxy, par un groupe ($C_1$-$C_6$)alkyle, par un ou deux groupes ($C_1$-$C_6$)alcoxy, par un groupe méthylènedioxy, par un groupe acétyle, par un groupe trifluorométhoxy ou par un groupe méthylthio,
R représente un atome d'hydrogène ou un groupe ($C_1$-$C_6$) alkyle,
étant toutefois exclus les composés de formule générale (I) dans laquelle X représente un groupe de formule CH, Y et Z représentent chacun un atome d'azote, et $R_1$ ou $R_2$ ne représente pas un groupe phényle éventuellement substitué.

Les composés ainsi exclus sont décrits dans le brevet US-5.478.939 comme agonistes muscariniques.

Schéma

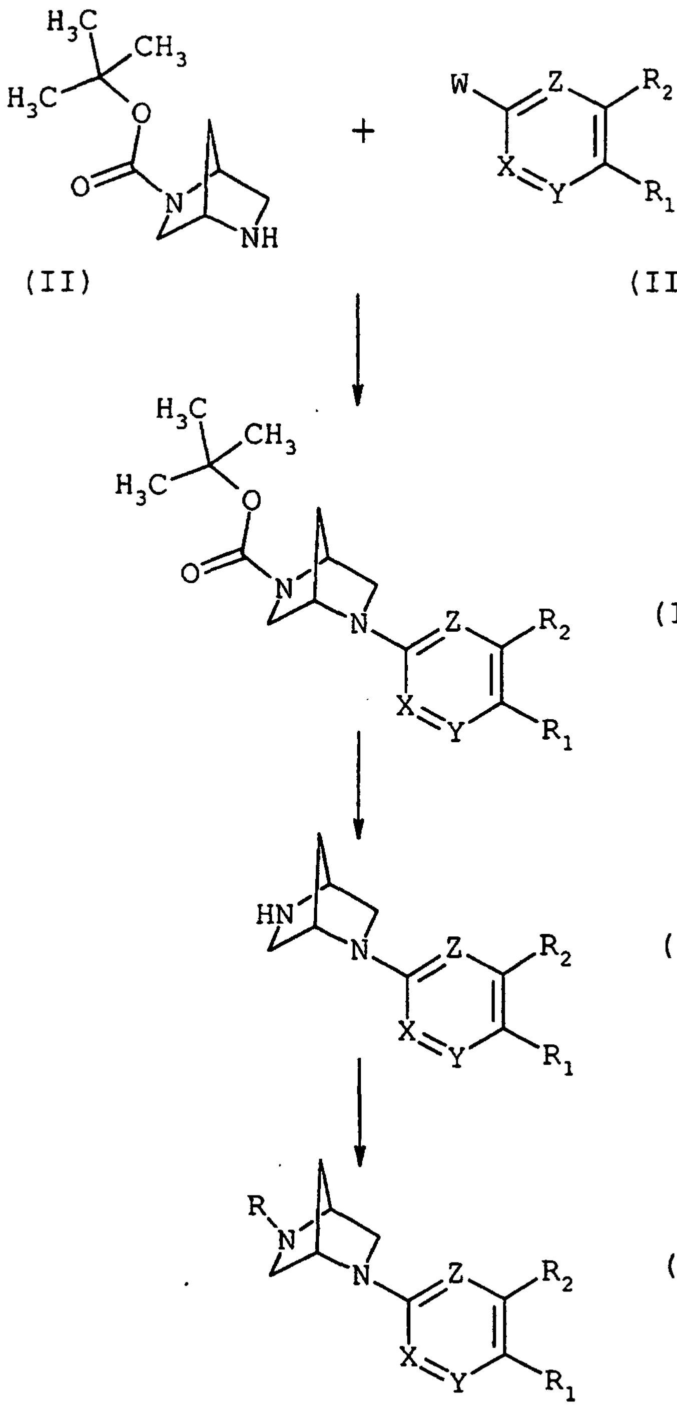

(II) (III)

(IV)

(Ia)

(I)

Les composés de l'invention peuvent exister à l'état de bases ou de sels d'addition à des acides. Ils peuvent aussi exister sous forme d'isomères (*S,S*) ou (*R,R*).

Les composés préférés sont ceux dans la formule desquels l'hétérocycle contenant X, Y et Z est un groupe pyridin-3-yle ou pyridazin-3-yle.

Conformément à l'invention, et selon le schéma qui précède, on peut préparer les composés de formule générale (I) en faisant réagir le 2,5-diazabicyclo[2.2.1]heptane-2-carboxylate de 1,1-diméthyléthyle de formule (II), avec un composé hétérocyclique de formule générale (III), dans laquelle X, Y, Z, $R_1$ et $R_2$ sont tels que définis ci-dessus et W représente un atome d'halogène.
On peut ainsi effectuer un couplage de Buchwald (*J. Org. Chem.* (1997) **62** 6066-6068) en présence d'un catalyseur au palladium tel que l'acétate de palladium, le tris(dibenzylidèneacétone)dipalladium(0), etc, d'un ligand de complexation tel que la triphénylphosphine, la tributylphosphine ou le 2,2'-bis(diphénylphosphino)-1,1'-binaphtyle, et d'une base, par exemple organique telle que le t-butoxyde de sodium, ou minérale telle que le carbonate de césium.

Lorsque X ou Z représente un atome d'azote on peut également effectuer un réaction de substitution nucléophile classique en présence d'une base forte telle que le carbonate de césium ou la triéthylamine.

On obtient un composé de formule générale (IV) dont on peut, si on le désire, modifier les substituants $R_1$ ou $R_2$ ; par exemple, lorsque $R_1$ ou $R_2$ représente un atome d'halogène, on peut lui substituer un groupe alkyle ou phényle par une réaction de Suzuki, au moyen d'acide alkylboronique ou phénylboronique, en présence de tétrakis(triphénylphosphino)palladium.

On déprotège ensuite le composé de formule générale (IV) de manière connue, par exemple au moyen d'acide trifluoroacétique ou chlorhydrique, pour obtenir un composé de formule générale (Ia), laquelle correspond à la formule générale (I) lorsque R représente un atome d'hydrogène.

On peut ensuite, si on le désire, effectuer une alkylation de ce composé, soit par une amination réductrice (formaldéhyde et cyanoborohydrure de sodium), soit par une réaction de Eschweiler-Clarke (formaldéhyde et acide formique).

Le (1*S*)-diazabicyclo[2.2.1]heptane-2-carboxylate de 1,1-diméthyléthyle de formule (II) est décrit dans *J. Org. Chem.* (1988) **53** 1580-1582 et le (1*R*)-diazabicyclo[2.2.1]-heptane-2-carboxylate de 1,1-diméthyléthyle de formule (II) est décrit dans la demande de brevet EP-400661.

Les composés de formule générale (III) sont disponibles dans le commerce ou sont accessibles par des méthodes décrites dans la littérature.

Les exemples qui vont suivre illustrent la préparation de quelques composés de l'invention. Les microanalyses élémentaires, et les spectres I.R. et R.M.N. confirment les structures des composés obtenus.
Les numéros indiqués entre parenthèses dans les titres des exemples correspondent à ceux de la lère colonne du tableau 1 donné plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

<u>Exemple 1</u> (Composé N°2).

Chlorhydrate de (1*S*)-2-(6-chloropyridin-3-yl)-2,5-diazabicyclo[2.2.1]heptane (2:1).

1.1. (1*S*)-2-(6-chloropyridin-3-yl)-2,5-diazabicyclo[2.2.1]heptane-5-carboxylate de 1,1-diméthyléthyle et (1*S*)-2-(5-bromopyridin-2-yl)-2,5-diazabicyclo[2.2.1]heptane-5-carboxylate de 1,1-diméthyléthyle.

Dans un ballon tricol de 500 ml on on introduit 4,96 g (25 mmoles) de (1*S*)-2,5-diazabicyclo[2.2.2]heptane-5-carboxylate de 1,1-diméthyléthyle, 5,0 g (26 mmoles) de 5-bromo-2-chloropyridine et 11,4 g (35 mmoles) de carbonate de césium en suspension dans 150 ml de tétrahydrofurane, on fait barboter un courant d'azote pendant 15 min, on ajoute 224 mg (1,0 mmole) d'acétate de palladium(II) et 623 mg (1,0 mmole) de 2,2'-bis(diphénylphosphino)-1,1'-binaphtyle et on chauffe le mélange au reflux pendant 22 h.
On le filtre, on évapore le solvant et on purifie le résidu par chromatographie sur colonne de gel de silice en éluant avec un mélange 40/60 d'acétate d'éthyle et d'heptane.
On obtient 1,63 g de (1*S*)-2-(5-bromopyridin-2-yl)-2,5-diazabicyclo[2.2.1]heptane-5-carboxylate de 1,1-diméthyléthyle, Point de fusion : 181-182°C, $[\alpha]_D^{20}$ = -229,4° (c=1, $CH_2Cl_2$), et 3,65 g de (1*S*)-2-(6-chloropyridin-3-yl)-2,5-diazabi_ cyclo [2.2.1]heptane-5-carboxylate de 1,1-diméthyléthyle, Point de fusion : 189°C, $[\alpha]_D^{20}$ = -224,5° (c=1, $CH_2Cl_2$).

1.2. Chlorhydrate de (1*S*)-2-(6-chloropyridin-3-yl)-2,5-diazabicyclo[2.2.1]heptane (2:1).

Dans un ballon tricol de 500 ml on dissout 3,0 g (9,71 mmoles) de (1*S*)-2-(6-chloropyridin-3-yl)-2,5-diazabicyclo[2.2.1]heptane-5-carboxylate de 1,1-diméthyléthyle dans 250 ml d'acétate d'éthyle et on fait barboter un courant d'acide chlorhydrique gazeux pendant 30 min.
On évapore le solvant sous pression réduite, on reprend le résidu avec 25 ml d'acétate d'éthyle, on recueille le solide par filtration et on le recristallise dans 25 ml d'éthanol.
On obtient 2,39 g de dichlorhydrate.
Point de fusion : 290-300°C, $[\alpha]_D^{20}$ = -105,6°, (c=0,5, $H_2O$).

Exemple 2 (Composé N°4)

(*E*)-But-2-ènedioate de (1*S*)-2-(6-chloropyridin-3-yl)-5-méthyl-2,5-diazabicyclo[2.2.1]heptane (2:1).

Dans un ballon tricol de 100 ml on introduit 0,55 g (2,65 mmoles) de (1*S*)-2-(6-chloropyridin-3-yl)-2,5-diazabicyclo[2.2.1]heptane en solution dans 20 ml d'éthanol, on refroidit la solution à 0°C, on ajoute lentement 0,43 ml (5,31 mmoles) d'une solution aqueuse de formaldéhyde à 37%, puis, par petites portions, 0,334 g (5,31 mmoles) de cyanoborohydrure de sodium, tout en maintenant la température proche de 0°C, et on maintient l'agitation pendant 30 min.
On dilue le mélange avec de l'eau, on l'extrait avec du chloroforme, on évapore la phase organique sous pression réduite et on purifie le résidu par chromatographie sur colonne de gel de silice en éluant avec un mélange 95/5/0,5 de dichlorométhane, méthanol et ammoniaque.
On obtient 0,290 g de composé sous forme de base.
On en dissout 0,258 g dans 20 ml d'éthanol, on traite la solution avec 0,134 g d'acide fumarique, on évapore le solvant sous pression réduite et on collecte le solide par filtration.
On isole finalement 0,292 g de fumarate.
Point de fusion : 143,6°C, $[\alpha]_D^{20}$ = -94,5° (c=0,5, $H_2O$).

Exemple 3 (Composé N°6).

Chlorhydrate de (1*S*)-2-(6-chloropyridazin-3-yl)-2,5-diazabicyclo[2.2.1]heptane (2:1).

3.1. (1*S*)-2-(6-chloropyridazin-3-yl)-2,5-diazabicyclo[2.2.1]heptane-5-carboxylate de 1,1-diméthyléthyle.

Dans un ballon tricol de 100 ml on introduit 0,397 g (2,0 mmoles) de (1*S*)-2,5-diazabicyclo[2.2.1]heptane-5-carboxylate de 1,1-diméthyléthyle, 0,328 g (2,2 mmoles) de 3,6-dichloropyridazine et 1,3 g (4 mmoles) de carbonate de césium dans 30 ml de toluène, et on chauffe le mélange au reflux pendant 48 h.
On le filtre, on évapore le solvant sous pression réduite, et on purifie le résidu par chromatographie sur colonne de gel de silice en éluant avec un mélange 80/20 d'acétate d'éthyle et d'heptane.
On obtient 0,3 g de composé.
Point de fusion : 198°C.

3.2. Chlorhydrate de (1*S*)-2-(6-chloropyridazin-3-yl)-2,5-diazabicyclo[2.2.1]heptane (2:1).

Dans un ballon tricol de 50 ml on introduit 0,25 g (0,933 mmole) de (1*S*)-2-(6-chloropyridazin-3-yl)-2,5-diazabicyclo[2.2.1]heptane-5-carboxylate de 1,1-diméthyléthyle et 20 ml d'acétate d'éthyle, on fait barboter de l'acide chlorhydrique gazeux pendant 10 min, et on maintient l'agitation pendant 30 min.
On évapore le solvant sous pression réduite, on reprend le résidu dans 5 ml d'acétate d'éthyle, on collecte le solide par filtration, on le rince à l'acétate d'éthyle et on le sèche.
On obtient 0,155 g de chlorhydrate.
Point de fusion : 260-270°C, $[\alpha]_D^{20}$ = -99,5° (c=0,5, $H_2O$).

Exemple 4 (Composé N°7).

(1*S*)-2-(6-Phénylpyridazin-3-yl)-2,5-diazabicyclo[2.2.1]heptane.

4.1. (1*S*)-2-(6-Phénylpyridazin-3-yl)-2,5-diazabicyclo[2.2.1]heptane-5-carboxylate de 1,1-diméthyléthyle.

Dans un ballon tricol de 100 ml on introduit 1,0 g (5,0 mmoles) de (1*S*)-2,5-diazabicyclo[2.2.1]heptane-2-carboxylate de 1,1-diméthyléthyle, 1,14 g (6,0 mmoles) de 3-chlo_ ro-6-phénylpyridazine et 0,84 ml (6,0 mmoles) de triéthylamine dans 45 ml de toluène et on chauffe au reflux pendant 72 h.
On dilue le milieu réactionnel à l'eau, on sépare la phase organique et on évapore le solvant sous pression réduite. On purifie le résidu par chromatographie sur gel de silice en éluant avec un mélange 90/10 de dichlorométhane et d'acétone. On obtient ainsi 0,96g de produit que l'on triture dans de l'éther diisopropylique pour isoler 0,92 g de produit pur après séchage.
Point de fusion : 208-209°C.

4.2. (1*S*)-2-(6-Phénylpyridazin-3-yl)-2,5-diazabicyclo[2.2.1]heptane.

Dans un ballon tricol de 100 ml on charge 0,9 g (2,55 mmoles) de (1*S*)-2-(6-phénylpyridazin-3-yl)-2,5-diazabicyclo[2.2.1]heptane-5-carboxylate de 1,1-diméthyléthyle et 25 ml d'acétate d'éthyle, on fait barboter de l'acide chlorhydrique gazeux pendant 10 min et on maintient l'agitation pendant 30 min.
On évapore le solvant sous pression réduite, on reprend le résidu dans une solution aqueuse d'hydroxyde de potassium et on extrait au chloroforme. On évapore la phase chloroformique et on triture le résidu obtenu dans de l'éther diisopropylique pour obtenir 0,59 g de produit pur.
Point de fusion : 162-163°C, $[\alpha]_D^{20}$ = -187,8° (c=0,5 $CHCl_3$).

<u>Exemple 5</u> (Composé N°8).

(1*S*)-2-(6-Phénylpyridazin-3-yl)-5-méthyl-2,5-diazabicyclo[2.2.1]heptane.

Dans un ballon tricol de 100 ml on introduit 0,439 g (1,74 mmole) de (1*S*)-2-(6-phénylpyridazin-3-yl)-2,5-diazabicyclo[2.2.1]heptane en solution dans 15 ml d'éthanol et 0,21 ml (3,48 mmoles) d'acide acétique. On ajoute 0,284 ml (3,48 mmoles) d'une solution de formaldéhyde aqueux, puis, par fractions, 0,218 g (3,48 mmoles) de cyanoborhydrure de sodium tout en maintenant la température autour de 5°C, et on maintient l'agitation pendant 1 h.
On dilue le milieu réactionnel avec de l'eau, on ajoute de la solution aqueuse d'hydroxyde de potassium et on extrait au chloroforme. On évapore la phase organique sous pression réduite et on purifie le résidu obtenu par chromatographie sur colonne de gel de silice en éluant avec un mélange de 97/3/0,3 de chloroforme, méthanol et ammoniaque.
On obtient ainsi 0,430 g de produit que l'on triture dans de l'éther diisopropylique à chaud pour isoler, après séchage, 0,392 g de composé.
Point de fusion : 112,6-113°C, $[\alpha]_D^{20}$ = -165,3° (c=0,5, $CHCl_3$)

<u>Exemple 6</u> (Composé N°9).

(1*S*)-2-(5-Phénylpyridin-3-yl)-2,5-diazabicyclo[2.2.1]heptane.

6.1. (1*S*)-2-(5-Bromopyridin-3-yl)-2,5-diazabicyclo[2.2.1]heptane-5-carboxylate de 1,1-diméthyléthyle.

Dans un ballon tricol de 250 ml on introduit 1,98 g (10 mmoles) de (1*S*)-2,5-diazabicyclo[2.2.1]heptane-2'-carboxylate de 1,1-diméthyléthyle, 7,1 g (30 mmoles) de 3,5-dibromopyridine et 4,56 g (14 mmoles) de carbonate de césium en suspension dans 100 ml de tétrahydrofurane, on fait barboter un courant d'azote pendant 15 min, on ajoute 90 mg (0,4 mmole) de diacétate de palladium et 249 mg (0,4 mmole) de 2,2'-bis(diphénylphosphino)-1,1'-binapthyle et on chauffe le mélange réactionnel au reflux pendant 22 h.
On filtre le milieu réactionnel et on concentre le filtrat sous pression réduite. On purifie le résidu obtenu par chromatographie sur gel de silice en éluant avec un mélange 40/60 d'acét&te d'éthyle et d'heptane.
On obtient ainsi 2,99 g de (1*S*)-2-(5-bromopyridin-3-yl)-2,5-diazabicyclo[2.2.1]heptane-5-carboxylate de 1,1-diméthyléthyle.
Point de fusion : 149°C, $[\alpha]_D^{20}$ = -196,5° (c=0,5,$CH_2Cl_2$).

6.2. (1*S*)-2-(5-Phénylpyridin-3-yl)-2,5-diazabicyclo_ [2.2.1]heptane-5-carboxylate de 1,1-diméthyléthyle.

Dans un ballon tricol de 25 ml on introduit, sous argon, 8 ml de benzène, 147 mg (0,127 mmole) de tétrakis (tri_ phénylphosphino)palladium et 1,5 g (4,23 mmoles) de (1*S*)-2-(5-bromopyridin-3-yl)-2,5-diazabicyclo[2.2.1]heptane-5-carboxylate de 1,1-diméthyléthyle, on ajoute 4 ml d'une solution aqueuse de carbonate de sodium 2 M, 0,568 g (4,66 mmoles) d'acide phénylboronique et 0,2 ml d'éthanol et on chauffe à reflux pendant 3h.
On décante le milieu réactionnel et on chromatographie la phase organique sur gel de silice en éluant avec un mélange 75/25 puis 90/10 d'acétate d'éthyle et d'heptane.
On obtient 1,55 g de composé que l'on triture dans de l'éther diisopropylique à chaud pour isoler 1,36 g de produit.
Point de fusion : 163,5-164°C, $[\alpha]_D^{20}$ = -194,9° (c=0,5,$CH_2Cl_2$)

6.3. (1*S*)-2-(5-Phénylpyridin-3-yl)-2,5-diazabicyclo[2.2.1]heptane.

Dans un ballon tricol de 100 ml on introduit 1,30 g (3,7 mmoles) de (1*S*)-2-(5-phénylpyridin-3-yl)-2,5-diazabicyclo[2.2.1]heptane-5-carboxylate de 1,1-diméthyléthyle dissous dans 40 ml d'acétate d'éthyle, on fait barboter de l'acide chlorhydrique gazeux pendant 30 min, puis on évapore le solvant.

On alcalinise le milieu réactionnel à l'ammoniaque et on extrait au chloroforme. On évapore le solvant et on triture le résidu dans de l'éther diéthylique.
On obtient ainsi 0,438 g de composé pur.
Point de fusion : 116-117°C, $[\alpha]_D^{20}$ = -134,9 (c=0,5,$CH_2Cl_2$).

Exemple 7 (Composé N°15).

(1*S*)-2-(6-Phénylpyridin-3-yl)-2,5-diazabicyclo[2.2.1]_ heptane.

7.1. 3-Bromo-6-phénylpyridine.

Dans un ballon tricol de 250 ml on introduit 10 g (42,2 mmoles) de 2,5-dibromopyridine, 5,2 g (42,2 mmoles) d'acide phénylboronique et 30 ml de benzène, on ajoute 1,5 g (1,3 mmole) de tétrakis(triphénylphosphino)palladium, 30 ml de benzène et 30 ml d'une solution aqueuse de carbonate de sodium 2M et 1,4 ml d'éthanol et on chauffe le mélange à reflux pendant 17 h.
On refroidit le milieu réactionnel, on le filtre, on décante la phase organique, on évapore le solvant sous pression réduite et on purifie le résidu par chromatographie sur gel de silice en éluant avec un mélange 70/30 d'heptane et de dichlorométhane.
On obtient 8,6 g de produit brut que l'on recristallise dans 7 ml d'éthanol. On obtient ainsi 5,6 g de produit pur sous forme de solide blanc.
Point de fusion : 69-72°C.

7.2. (1*S*)-2-(6-Phénylpyridin-3-yl)-2,5-diazabicyclo[2.2.1]heptane-5-carboxylate de 1,1-diméthyléthyle.

Dans un ballon tricol de 100 ml on introduit, sous azote, 40 ml de tétrahydrofurane, 1,59 g (8,0 mmoles) de (1*S*)-2,5-diazabicyclo[2.2.1]heptane-2-carboxylate de 1,1-diméthyléthyle, 2,25 g (9,6 mmoles) de 3-bromo-6-phénylpyridine, 3,65 g (11,2 mmoles) de carbonate de césium, 72 mg de bisacétate de palladium, et 0,20 g (0,32 mmole) de 2,2'-bis(diphénylphosphino)-1,1'-binapthyle et on chauffe le mélange à reflux pendant 18h.
On le filtre, on évapore le solvant sous pression réduite et on purifie le résidu par chromatographie sur gel de silice en éluant avec un mélange 40/60 d'acétate d'éthyle et d'heptane pour obtenir 2,9 g de produit brut que l'on triture dans de l'éther diisopropylique.
On obtient ainsi 2,4g de produit pur.
Point de fusion : 147,5°C, $[\alpha]_D^{20}$ = -251,3 (c=0,5,$CH_2Cl_2$).

7.3. (1*S*)-2-(6-Phénylpyridin-3-yl)-2,5-diazabicyclo_ [2.2.1]heptane.

Dans un ballon tricol de 250 ml on introduit 2,3 g (6,8 mmoles) de (1*S*)-2-(6-phénylpyridin-3-yl)-2,5-diazabicyclo[2.2.1]heptane-5-carboxylate de 1,1-diméthyléthyle en solution dans 100 ml d'acétate d'éthyle et on fait barboter de l'acide chlorhydrique gazeux pendant 30 min.
On évapore le solvant sous pression réduite et on triture le résidu dans de l'éther. On alcalinise le résidu par l'addition d'une solution aqueuse d'hydroxyde de sodium et on extrait au chloroforme. On évapore le solvant sous pression réduite pour obtenir 1,7 g de solide que l'on triture dans de l'éther diisopropylique. On obtient ainsi 1,52 g de produit sous forme de solide blanc.
Point de fusion : 121,5-122°C, $[\alpha]_D^{20}$ = -178,7° (c=0,5,$CH_2Cl_2$)

Le tableau qui suit illustre les structures chimiques et les propriétés physiques de quelques composés de l'invention. Dans la colonne "Sel", "-" désigne un composé à l'état de base, HBr désigne un bromhydrate, "HCl" désigne un chlorhydrate, "Hbr désigne un bromhydrate", "fum" désigne un fumarate, ou (*E*)-But-2-ènedioate, "ox" désigne un oxalate, ou éthanedioate et "TFA" désigne un trifluoroacétate.
* Dans la dernière colonne, les pouvoirs rotatoires des composés sous forme de sels sont donnés pour (c=0,5, $H_2O$), les pouvoirs rotatoires des composés 3, 7, 8, 9, et 11 sont donnés pour (c=0,5, $CHCl_3$), les pouvoirs rotatoires des composés 15 et 16 sont donnés pour (c=0,5, $CH_2Cl_2$) et les pouvoirs rotatoires des composés 18 à 36 sont donnés pour (c=0,5, $CH_3OH$).

Tableau

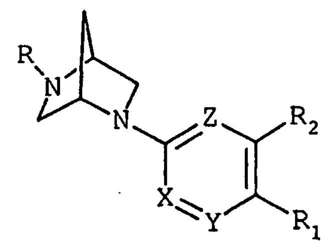

(I)

| N° | X | Y | Z | R | $R_1$ | $R_2$ | Sel | F (°C) | $[\alpha]_D^{20}$ (*) |
|---|---|---|---|---|---|---|---|---|---|
| 1 | CH | N | CH | H | H | H | HBr 2:1 | 268-270 | -103,2° |
| 2 | CH | N | CH | H | Cl | H | HCl 2:1 | 290-300 | -105,6° |
| 3 | CH | N | CH | $CH_3$ | H | H | - | huile | -126,7° |
| 4 | CH | N | CH | $CH_3$ | Cl | H | fum 1:1 | 143,6 | -94,5° |
| 5 | N | CH | CH | H | Br | H | HCl 2:1 | 215-220 | -99,5° |
| 6 | N | N | CH | H | Cl | H | HCl 2:1 | 260-270 | -99,5° |
| 7 | N | N | CH | H | $C_6H_5$ | H | - | 162-163 | -187,8 |
| 8 | N | N | CH | $CH_3$ | $C_6H_5$ | H | - | 112,6-113 | -165,3 |
| 9 | CH | N | CH | H | H | $C_6H_5$ | - | 116-117 | -134,9 |
| 10 | N | CH | CH | H | $C_6H_5$ | H | HCl 2:1 | 192-205 | -108,1 |
| 11 | N | CH | CH | $CH_3$ | $C_6H_5$ | H | - | 45-50 | -170,4 |
| 12 | CH | N | CH | H | $OCH_3$ | H | HCl 2:1 | 188-190 | -85,7 |
| 13 | CH | N | CH | H | Br | H | fum 1:1 | 199-202 | -81,8 |

| N° | X | Y | Z | R | $R_1$ | $R_2$ | Sel | F (°C) | $[\alpha]_D^{20}$ (*) |
|---|---|---|---|---|---|---|---|---|---|
| 14 | CH | N | CH | H | H | Br | fum 1:1 | 225-230 | -76,5 |
| 15 | CH | N | CH | H | $C_6H_5$ | H | - | 121,5-122 | -178,7 |
| 16 | CH | N | CH | $CH_3$ | $C_6H_5$ | H | - | 86,5-87 | -174,1 |
| 17 | CH | N | CH | $CH_3$ | H | $C_6H_5$ | ox 2:1 | 191-192 | -83,2 |
| 18 | CH | N | CH | H | H | 3,4-$OCH_2O$-$C_6H_3$ | TFA 2:1 | 150-151 | -68,5 |
| 19 | CH | N | CH | H | H | 3,5-$(CF_3)_2$-$C_6H_3$ | TFA 2:1 | 176-177 | -61,3 |
| 20 | CH | N | CH | H | H | 3-Cl-$C_6H_4$ | HBr 3:1 | 280 | -70,5 |
| 21 | CH | N | CH | H | H | 2,4-$Cl_2$-$C_6H_3$ | TFA 2:1 | 128-129 | -63,0 |
| 22 | CH | N | CH | H | H | 4-$CH_3O$-$C_6H_4$ | TFA 2:1 | 127-128 | -69,0 |
| 23 | CH | N | CH | H | H | 4-$CF_3$-$C_6H_4$ | TFA 2:1 | 189-190 | -65,0 |
| 24 | CH | N | CH | H | H | 4-$CH_3$-$C_6H_4$ | TFA 2:1 | 160-161 | -71,5 |
| 25 | CH | N | CH | H | H | 3-Cl,4-F-$C_6H_3$ | TFA 2:1 | 150 | -67,7 |
| 26 | CH | N | CH | H | H | 4-F-$C_6H_4$ | HBr 2:1 | >280 | -76,2 |
| 27 | CH | N | CH | H | H | 3-$CH_3CO$-$C_6H_4$ | HBr 2:1 | >280 | -79,1 |
| 28 | CH | N | CH | H | H | 2-$CH_3$-$C_6H_4$ | TFA 2:1 | 157 | -70,8 |
| 29 | CH | N | CH | H | H | 2-$CH_3O$-$C_6H_4$ | TFA 2:1 | 134 | -64,7 |
| 30 | CH | N | CH | H | H | 2-Cl-$C_6H_4$ | TFA 2:1 | 139-140 | -68,9 |
| 31 | CH | N | CH | H | H | 2,3-$Cl_2$-$C_6H_3$ | TFA 2:1 | 146-147 | -66,5 |
| 32 | CH | N | CH | H | H | 3,4-$Cl_2$-$C_6H_3$ | TFA 2:1 | 140 | -66,9 |
| 33 | CH | N | CH | H | H | 3-$CF_3$-$C_6H_4$ | TFA 2:1 | 126 | -70,2 |

| N° | X | Y | Z | R | $R_1$ | $R_2$ | Sel | F (°C) | $[\alpha]_D^{20}$ (*) |
|---|---|---|---|---|---|---|---|---|---|
| 34 | CH | N | CH | H | H | $3-CH_3O-C_6H_4$ | HBr 2:1 | 235 | -76,3 |
| 35 | CH | N | CH | H | H | $4-CF_3O-C_6H_4$ | TFA 2:1 | 165-166 | -63,0 |
| 36 | CH | N | CH | H | H | $4-CH_3S-C_6H_4$ | TFA 2:1 | 113 | -66,7 |
| 37 | CH | N | CH | $CH_3$ | $4-F-C_6H_4$ | H | HBr 2:1 | 175-176 | -101,6 |
| 38 | CH | N | CH | $CH_3$ | $4-CH_3O-C_6H_4$ | H | ox 2:1 | 193-194 | -102,0 |
| 39 | CH | N | CH | $CH_3$ | $3-CH_3-C_6H_4$ | H | ox 2:1 | 172-173 | -68,2 |
| 40 | CH | N | CH | $CH_3$ | $3-CH_3CO-C_6H_4$ | H | HBr 2:1 | 320-321 | -94,2 |
| 41 | CH | N | CH | $CH_3$ | $4-CF_3-C_6H_4$ | H | ox 2:1 | 134-135 | -82,2 |
| 42 | CH | N | CH | $CH_3$ | $3,4-(CH_3O)_2-C_6H_3$ | H | ox 2:1 | 179-180 | -86,0 |
| 43 | CH | N | CH | $CH_3$ | $2,4-(CH_3O)_2-C_6H_3$ | H | HBr 2:1 | 196-197 | -65,4 |
| 44 | CH | N | CH | $CH_3$ | $3-CH_3O-C_6H_4$ | H | HBr 2:1 | 255-256 | -109,8 |
| 45 | CH | N | CH | $CH_3$ | $3,4-OCH_2O-C_6H_3$ | H | ox 2:1 | 163-164 | -93,5 |
| 46 | CH | N | CH | $CH_3$ | $3-F-C_6H_4$ | H | ox 2:1 | 140-141 | -75,6 |
| 47 | CH | N | CH | $CH_3$ | $4-CH_3CH_2-C_6H_4$ | H | ox 2:1 | 185-186 | -87,6 |
| 48 | CH | N | CH | $CH_3$ | $4-F,3-Cl-C_6H_3$ | H | ox 2:1 | 154-155 | -69,4 |
| 49 | CH | N | CH | $CH_3$ | $2-CH_3O-C_6H_4$ | H | - | huile | -51,2 |
| 50 | CH | N | CH | $CH_3$ | $3-CF_3-C_6H_4$ | H | - | huile | -62,0 |
| 51 | CH | N | CH | $CH_3$ | $4-CH_3-C_6H_4$ | H | - | huile | -35,6 |

Les composés de l'invention ont fait l'objet d'essais qui ont mis en évidence leurs propriétés thérapeutiques.

Ainsi ils ont été étudiés quant à leur affinité vis-à-vis des récepteurs nicotiniques contenant la sous-unité $\alpha_4\beta_2$ selon les méthodes décrites par Anderson et Arneric, Eur. *J. Pharmacol* (1994) **253** 261, et par Hall et coll., *Brain Res.* (1993) **600** 127.

On décapite des rats mâles Sprague Dawley de 150 à 200 g et on prélève rapidement la totalité du cerveau, on l'homogénéise dans 15 volumes d'une solution de sucrose à 0,32 M à 4°C puis on le centrifuge à 1000 g pendant 10 min. On élimine le culot, et on centrifuge le surnageant à 20000 g pendant 20 min à 4°C. On récupère le culot et on l'homogénéise à l'aide d'un broyeur Polytron™ dans 15. volumes d'eau bidistillée à 4°C, puis on le centrifuge à 8000 g pendant 20 min. On élimine le culot et on centrifuge le surnageant et la couche de peau ("buffy coat") à 40000 g pendant 20 min, on récupère le culot, on le remet en suspension dans 15 ml d'eau bidistillée à 4°C et on le centrifuge encore une fois à 40000 g avant de le conserver à -80°C.

Le jour de l'expérience on décongèle lentement le tissu et on le met en suspension dans 3 volumes de tampon. On fait incuber 150 µl de cette suspension membranaire à 4°C pendant 120 min en présence de 100 µl de [$^3$H]cytisine 1 nM dans un volume final de 500 µl de tampon, en présence ou en absence de composé à tester. On arrête la réaction par filtration sur des filtres Whatman GF/B™ préalablement traités avec de la polyéthylèneimine, on rince les filtres avec deux fois 5 ml de tampon à 4°C, et on mesure la radioactivité retenue sur le filtre par scintigraphie liquide.On détermine la liaison non spécifique en présence de (-)-nicotine à 10 µM ; la liaison non spécifique représente 75 à 85% de la liaison totale récupérée sur le filtre. Pour chaque concentration de composé étudié on détermine le pourcentage d'inhibition de la liaison spécifique de [$^3$H]cytisine, puis on calcule la $CI_{50}$, concentration de composé qui inhibe 50% de la liaison spécifique. Les $CI_{50}$ des composés de l'invention les plus affins se situent entre 0,001 et 0,25 µM.

Les composés de l'invention ont aussi été étudiés quant à leur affinité vis-à-vis des récepteurs nicotiniques contenant la sous-unité $\alpha$7, selon les méthodes décrites par Marks et Collins, *J. Pharmacol . Exp. Ther.* (1982) **22** 554 et Marks et al., *Mol. Pharmacol*. (1986) **30** 427.

On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4°C, puis on le centrifuge à 1000 g pendant 10 min. On élimine le culot, et on centrifuge le surnageant à 8000 g pendant 20 min à 4°C. On récupère le culot et on l'homogénéise à l'aide d'un broyeur Polytron™ dans 15 volumes d'eau bidistillée à 4°C, puis on le centifuge à 8000 g pendant 20 min. On élimine le culot et on centrifuge le surnageant et la couche de peau ("buffy coat") à 40000 g pendant 20 min. On récupère le culot, on le remet en suspension avec 15 volumes d'eau bidistillée à 4°C et on le centrifuge encore une fois à 40000 g pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis le membranes sont incubées pendant 60 min à 37°C, à l'obscurité, en présence de 50 µl de [$^3$H]$\alpha$-bungarotoxine 1 nM dans un volume final de 250 µl de tampon HEPES 20 mM à 0,05% de polyéthylèneimine. On arrête la réaction par filtration sur des filtres Whatman GF/C™ préalablement traités pendant 3 heures avec de la polyéthylèneimine à 0,5%. On rince les filtres avec deux fois 5 ml de tampon à 4°C, et on mesure la radioactivité retenue sur chaque filtre par scintigraphie liquide. On détermine la liaison non spécifique en présence de $\alpha$-bungarotoxine à 1 µM final ; la liaison non spécifique représente environ 60% de la liaison totale récupérée sur le filtre. Pour chaque concentration de composé étudié on détermine le pourcentage d'inhibition de la liaison spécifique de [$^3$H]$\alpha$-bungarotoxine, puis on calcule la $CI_{50}$, concentration de composé qui inhibe 50% de la liaison spécifique.

Les $CI_{50}$ des composés de l'invention les plus affins se situent entre 0,005 et 0,6 µM.

Les composés de l'invention ont également été étudiés quant à leur affinité vis-à-vis des récepteurs nicotiniques périphérique de type ganglionnaire selon la méthode décrite par Houghtling et al., *Mol. Pharmacol.* (1995) **48** 280-287. La capacité d'un composé à déplacer la [$^3$H]-epibatidine des membranes de glandes surrénales de boeuf mesure son affinité pour ce récepteur.

On décongèle des glandes surrénales de boeuf conservées à -80°C, et on les homogénéise à l'aide d'un broyeur Polytron™ dans 20 volumes de tampon Tris-HCl 50 mM à pH 7,4 à 4°C, puis on les centifuge à 35000 g pendant 10 min. On élimine le surnageant et on remet le culot en suspension dans 30 volumes de tampon Tris-HCl 50 mM à 4°C et on réhomogénéise avant de recentrifuger à 35000 g pendant 10 min. On reprend le dernier culot dans 10 volumes de tampon Tris-HCl à 4°C. On fait incuber 100 µl de membrane soit 10 mg de tissu frais à 24°C pendant 3 h en présence de 50 µl de [$^3$H]-epibatidine 0,66 nM final dans un volume final de 250 µl de tampon, en présence ou en absence de composé à tester. On arrête la réaction par dilution des échantillons avec du tampon Tris-HCl 50 µM pH 7,4 à 4°C puis on filtre sur de filtres Whatman GF/C™ préalablement traités pendant 3 heures avec de la polyéthylèneimine à 0,5%. On rince les filtres 2 fois par 5 ml de tampon et on mesure la radioactivité retenue sur le filtre par scintigraphie liquide. On détermine la liaison non spécifique en présence de (-)nicotine 2 mM final ; la liaison non spécifique représente 30 à 40% de la liaison totale récupérée sur le filtre. Pour chaque concentration de composé étudié on détermine le pourcentage d'inhibition de la liaison spécifique de [$^3$H]-epibatidine, puis on calcule la $CI_{50}$, concentration de composé qui inhibe 50% de la liaison spécifique.

Les $CI_{50}$ des composés de l'invention se situent entre 0,1 et 20 µM.

Les résultats des essais qui précèdent montrent que certains composés de l'invention sont des ligands sélectifs pour les sous-unités $\alpha_4\beta_2$, $\alpha_7$ ou $\alpha_3$ du récepteur nicotinique et que d'autres sont mixtes $\alpha_4\beta_2$ et $\alpha_7$, $\alpha_4\beta_2$ et $\alpha_3$, ou $\alpha_7$ et $\alpha_3$.

Enfin les composés de l'invention ont fait l'objet d'essais qui ont mis en évidence leurs propriétés analgésiques. Ainsi ils ont été étudiés dans le modèle de la plaque chauffante, selon la méthode de Eddy et Leimbach, *J. Pharmacol. Exp. Ther.* (1953) **107** 385-393 dans le but de rechercher et quantifier un éventuel effet analgésique.

Des souris de 20 à 30 g sont soumis à un stimulus thermique par contact des pattes avec une plaque maintenue à la température constante de 57,5°C par un bain-marie thermostaté. On mesure le temps de réaction à la douleur qui se manifeste par un lèchage de pattes ou un saut.

Ainsi, après le délai de prétraitement effectué par voie sous-cutanée ou orale (chaque lot étant constitué de huit animaux pour un même prétraitement), les souris sont déposées individuellement sur la plaque et le temps de réaction à la douleur est mesuré. L'animal est retiré de la plaque immédiatement après la manifestation de la douleur. Le temps maximum d'exposition au stimulus est de 30 secondes.

On exprime pour chaque lot le temps moyen de réaction accompagné de l'erreur standard à la moyenne (e.s.m). Une analyse de variance non paramétrique (Kruskal-Wallis), est effectuée sur l'ensemble du lot. Un test de Wilcoxon permet la comparaison de chaque lot traité au lot témoin. Les différences sont considérées comme statistiquement significatives au seuil 5%.

Ce temps de réaction est significativement augmenté par les analgésiques principalement à effets centraux.

Les composés de l'invention montrent une activité dans ce test aux doses comprises entre 0,3 et 30 mg/kg par voie intrapéritonéale ou orale.

Ces résultats suggèrent l'utilisation des composés dans le traitement ou la prévention des désordres liés à un dysfonctionnement des récepteurs nicotiniques, notamment au niveau du système nerveux central ou du système gastro-intestinal.

Au niveau du système nerveux central ces désordres comprennent les altérations cognitives, plus spécifiquement mnésiques, mais également attentionnelles, liées à la maladie d'Alzheimer, au vieillissement pathologique (Age Associated Memory Impairment, AAMI), au syndrome Parkinsonien, à la trisomie 21 (Down's syndrome), au syndrome alcoolique de Korsakoff, aux démences vasculaires (multi-infarct dementia, MID) et au déficit de l'attention/hyperactivité (attention deficit/hyperactivity disorder, ADHD).

Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.

Les composés de l'invention peuvent également constituer un traitement curatif ou symptomatique des accidents et traumatismes crâniens ou médullaires, des accidents vasculaires cérébraux et des épisodes hypoxiques cérébraux, ainsi que des autres maladies neurodégénératives aiguës ou chroniques.

Ils peuvent être utilisés dans les cas de pathologies psychiatriques : schizophrénie, dépression, anxiété, attaques de panique, comportements compulsifs et obsessionnels.

Ils peuvent prévenir les symptômes dus au sevrage au tabac, à l'alcool, aux différentes substances induisant une dépendance, telles que cocaïne, LSD, cannabis, benzodiazépines.

Enfin ils peuvent être utiles pour le traitement de la douleur aiguë et neuropathique.

Au niveau du système gastro-intestinal les composés de l'invention pourraient être utiles dans le traitement de la maladie de Crohn, de la colite ulcéreuse, du syndrome du côlon irritable et de l'obésité.

A cet effet les composés de l'invention peuvent être présentés sous toutes formes de compositions appropriées à l'administration entérale, parentérale ou transdermique, telles que comprimés, dragées, gélules, capsules, suspensions ou solutions buvables ou injectables telles que sirops ou ampoules, timbres transdermiques ("patch"), etc, associés à des excipients convenables, et dosés pour permettre une administration journalière de 0,01 à 20 mg/kg.

## Revendications

1. Composé répondant à la formule générale (I)

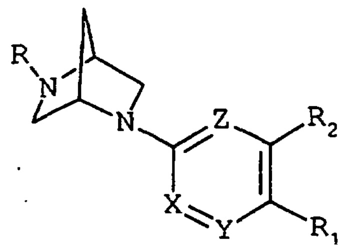

(I)

dans laquelle

l'un des symboles X, Y et Z représente un atome d'azote, un autre représente un groupe de formule C-$R_3$ et le troisième représente un atome d'azote ou un groupe de formule C-$R_4$,

$R_3$ et $R_4$ représentent chacun, indépendamment l'un de l'autre, un atome d'hydrogène ou d'halogène ou un groupe trifluorométhyle, cyano, hydroxy, ($C_1$-$C_6$)alkyle ou ($C_1$-$C_6$)alcoxy,

$R_1$ et $R_2$ représentent chacun, indépendamment l'un de l'autre, un atome d'hydrogène ou d'halogène ou un groupe trifluorométhyle, cyano, hydroxy, ($C_1$-$C_6$)alkyle, ($C_1$-$C_6$)-alcoxy, ou phényle éventuellement substitué par un ou deux atomes d'halogènes, par un ou deux groupes trifluorométhyle, par un groupe cyano, par un groupe nitro, par un groupe hydroxy, par un groupe ($C_1$-$C_6$)alkyle, par un ou deux groupes ($C_1$-$C_6$)alcoxy, par un groupe méthylènedioxy, par un groupe acétyle, par un groupe trifluorométhoxy ou par un groupe méthylthio,

R représente un atome d'hydrogène ou un groupe ($C_1$-$C_6$) alkyle,

étant exclus les composés de formule générale (I) dans laquelle X représente un groupe de formule CH, Y et Z représentent chacun un atome d'azote, et $R_1$ ou $R_2$ ne représente pas un groupe phényle éventuellement substitué, à l'état de base ou de sel d'addition à un acide.

**2.** Composé selon la revendication 1, **caractérisé en ce que** l'hétérocycle contenant X, Y et Z est un groupe pyridin-3-yle.

**3.** Composé selon la revendication 1, **caractérisé en ce que** l'hétérocycle contenant X, Y et Z est un groupe pyridazin-3-yle.

**4.** Médicament **caractérisé en ce qu'**il consiste en un composé selon l'une des revendication 1 à 3.

**5.** Composition pharmaceutique **caractérisée en ce qu'**elle contient un composé selon l'une des revendications 1 à 3, associé à un excipient.

## Patentansprüche

**1.** Verbindung der allgemeinen Formel (I)

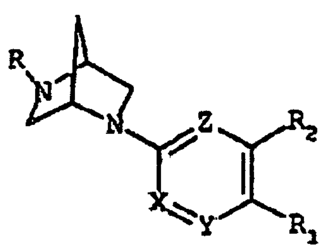

(I)

in der

eines der Symbole X, Y und Z ein Stickstoffatom, ein weiteres eine Gruppe der Formel C-$R_3$ und das dritte ein Stickstoffatom oder eine Gruppe der Formel C-$R_4$,

worin $R_3$ und $R_4$ jeweils unabhängig voneinander ein Wasserstoffatom oder ein Halogenatom oder eine Trifluormethylgruppe, Cyanogruppe, Hydroxygruppe, ($C_1$-$C_6$)-Alkylgruppe oder ($C_1$-$C_6$)-Alkoxygruppe darstellen, bedeuten,

$R_1$ und $R_2$ jeweils unabhängig voneinander ein Wasserstoffatom oder ein Halogenatom oder eine Trifluormethylgruppe, Cyanogruppe, Hydroxygruppe, ($C_1$-$C_6$)-Alkylgruppe, ($C_1$-$C_6$)-Alkoxygruppe oder Phenylgruppe, die gegebenenfalls durch ein oder zwei Halogenatome, durch eine oder zwei Trifluormethylgruppen, durch eine Cyanogruppe, durch eine Nitrogruppe, durch eine Hydroxygruppe, durch eine ($C_1$-$C_6$)-Alkylgruppe, durch eine oder zwei ($C_1$-$C_6$)-Alkoxygruppen, durch eine Methylendioxygruppe, durch eine Acetylgruppe, durch eine Trifluormethoxygruppe oder durch eine Methylthiogruppe substituiert ist,
R ein Wasserstoffatom oder eine ($C_1$-$C_6$)-Alkylgruppe bedeutet,
wobei die Verbindungen der allgemeinen Formel (I) ausgeschlossen sind, in der X eine Gruppe der Formel CH, Y und Z jeweils ein Stickstoffatom und $R_1$ oder $R_2$ nicht gegebenenfalls substituierte Phenylgruppe bedeuten, in Form der Base oder des Säureadditionssalzes.

2. Verbindung nach Anspruch 1, **dadurch gekennzeichnet, daß** der X, Y und Z enthaltende Heterocyclus eine Pyridin-3-yl-gruppe ist.

3. Verbindung nach Anspruch 1, **dadurch gekennzeichnet, daß** der X, Y und Z enthaltende Heterocyclus eine Pyridazin-3-yl-gruppe ist.

4. Arzneimittel, **dadurch gekennzeichnet, daß** es aus einer Verbindung nach einem der Ansprüche 1 bis 3 besteht.

5. Pharmazeutische Zubereitung, **dadurch gekennzeichnet, daß** sie eine Verbindung nach einem der Ansprüche 1 bis 3 in Kombination mit einem Trägermatrial enthält.

## Claims

1. Compound corresponding to the general formula (I)

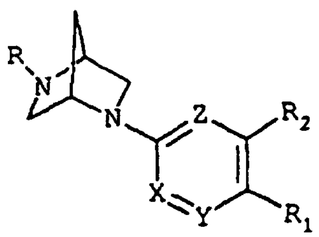

(I)

in which
one of the symbols X, Y and Z represents a nitrogen atom, another represents a group of formula C-$R_3$ and the third represents a nitrogen atom or a group of formula C-$R_4$,
$R_3$ and $R_4$ represent, independently of each other, a hydrogen or halogen atom or a trifluoromethyl, cyano, hydroxyl, ($C_1$-$C_6$)alkyl or ($C_1$-$C_6$)alkoxy group,
$R_1$ and $R_2$ represent, independently of each other, a hydrogen or halogen atom or a trifluoromethyl, cyano, hydroxyl, ($C_1$-$C_6$)alkyl or ($C_1$-$C_6$)alkoxy group, or a phenyl group optionally substituted with one or two halogen atoms, with one or two trifluoromethyl groups, with a cyano group, with a nitro group, with a hydroxyl group, with a ($C_1$-$C_6$)alkyl group, with one or two ($C_1$-$C_6$)alkoxy groups, with a methylenedioxy group, with an acetyl group, with a trifluoromethoxy group or with a methylthio group,
R represents a hydrogen atom or a ($C_1$-$C_6$)alkyl group,
with the exclusion of the compounds of general formula (I) in which X represents a group of formula CH, Y and Z each represent a nitrogen atom, and $R_1$ or $R_2$ does not represent an optionally substituted phenyl group, in base form or in the form of an addition salt with an acid.

2. Compound according to Claim 1, **characterized in that** the heterocycle containing X, Y and Z is a 3-pyridyl group.

3. Compound according to Claim 1, **characterized in that** the heterocycle containing X, Y and Z is a 3-pyridazinyl group.

4. Medicinal product, **characterized in that** it consists of a compound according to one of Claims 1 to 3.

5. Pharmaceutical composition, **characterized in that** it contains a compound according to one of Claims 1 to 3,

combined with an excipient.